# EUROPEAN PATENT SPECIFICATION

(11) **EP 3 403 687 B1**
(45) Date of publication and mention of the grant of the patent: **25.08.2021**
(21) Application number: 18183128.0
(22) Date of filing: 02.06.2011
(51) Int. Cl.: A61M 37/00, A61M 5/158, A45D 44/00, A61M 5/42, A61M 5/46, A61M 5/32, A61B 17/20, A61M 25/00, A61B 17/00

(54) **MODULE FOR USE IN SKIN SURGERY**
MODUL ZUR VERWENDUNG IN DER HAUTCHIRURGIE
MODULE POUR UTILISATION DANS LA CHIRURGIE DE LA PEAU

(30) Priority: 28.10.2010 KR 20100105995
(43) Date of publication of application: 21.11.2018
(62) Divisional of application: 11836517.0
(73) Proprietor: Huons Medical Co., Ltd., Seongnam-si, Gyeonggi-do (KR)
(72) Inventor: BANG, Si-Yeol, Yongin-si Gyeonggi-do (KR)
(74) Representative: Manitz Finsterwald Patent- und Rechtsanwaltspartnerschaft mbB

(56) References cited:
- WO-A2-2005/000382
- WO-A2-2009/107945
- KR-A- 20100 015 018
- KR-A- 20100 060 222
- KR-A- 20100 083 548
- KR-B1- 100 902 133

## Description

### [Technical Field]

The present invention relates to a module for use in skin surgery, and more particularly, to a module for use in skin surgery, which can accurately control a depth that drug is injected into the skin by a simple manipulation.

### [Background Art]

In general, multi-hole therapy is a therapy for making a plurality of fine holes into the skin and injecting drug into a human body through the holes, and is applicable to various fields, for instance, treatment of various skin troubles, such as wrinkle, freckles, blemishes, stretch marks, pimples, acnes, pigmentation, and so on, hair-conditioning, hair loss treatment, obesity treatment, etc.

Such a multi-hole therapy can obtain a rapid surgical effect by granulating using propagation ability of damaged skin tissues or by interfusing drug or the like through fine holes.

However, many of aestheticians repeat an action to fix needles into the skin and unfix the needles one by one depending on his or her intuition, and hence, the multi-hole therapy has a problem in that a subject may complain of pain or drug does not uniformly pass through the skin because the needles are not injected to a predetermined depth.

Moreover, in the case that the multi-hole therapy is applied to the middle aged who are losing the skin's elasticity, even though the aesthetician wants to perforate holes to a wanted depth, it is difficult to retain proper tension of the skin while a plurality of the holes are perforated into the skin.

That is, the multi-hole therapy has a problem in that it causes pain because surgical operation tools such as needles cannot exactly perforate holes into wanted positions of the skin and slip or are pushed in a state where the needles are stuck in the skin during the process of perforating a plurality of the holes into the skin.

Examples of devices capable of simultaneously perforating multiple holes in the skin are disclosed in WO 2005/000382 A2 and KR 2010 0015018 A.

### [Disclosure]

### [Technical Problem]

Accordingly, the present invention has been made in an effort to solve the above-mentioned problems occurring in the prior arts, and it is an object of the present invention to provide a module for use in skin surgery, which can accurately control a depth that drug is injected into the skin by a simple manipulation.

It is another object of the present invention to provide a module for use in skin surgery, which can accurately perforate the skin while providing a predetermined tension to the skin during the skin surgery.

### [Technical Solution]

To achieve the above objects, the present invention provides a module for use in skin surgery comprising the features of claim 1.

### [Advantageous Effects]

As described above, the module for use in skin surgery has following advantages as follows.

First, the present invention can accurately regulate the depth of the needles inserted into the skin by a simple manipulation that the needles of the needle assembly are extended and contracted by the reciprocating assembly, which is screw-coupled inside the main body and moves forward and backward according to the forward and backward rotation, or by the rotation assembly, which moves the reciprocating assembly forward and backward by a rotary motion.

Furthermore, because one end portion of the main body is closed, in the case that a device for providing vibration using a driving force is connected to the main body, the closed end portion of the main body provides proper tension while getting in contact with the skin, and hence, the present invention minimize pain and enables the user to carry out the skin surgery smoothly.

Additionally, the present invention can adopt the needle assembly, which has the solid core needles, or the needle assembly, which has the needles opened at both ends so as to simultaneously inject drug received from the drug injection hole into the skin while perforating fine holes into the skin, according to the subjects to undergo the skin surgery and used devices, and hence, the present invention is versatile-applicable.

### [Description of Drawings]

FIG. 1 is a perspective view showing an outward appearance of a module for use in skin surgery according to an embodiment which is not claimed.
FIG. 2 is a sectional view taken along the line A-A' of FIG. 1.
FIG. 3 is an enlarged view of a B part of FIG. 2.
FIG. 4 is a view of another example of a needle assembly shown according to the cross section taken along the line A-A' of FIG. 1.
FIG. 5 is a sectional conceptual view showing the entire structure of a module for use in skin surgery according to a preferred embodiment of the present invention.
FIG. 6 is a sectional view taken along the line C-C' of FIG. 5.
FIG. 7 is a sectional conceptual view showing another example of FIG. 5.
FIG. 8 is a sectional conceptual view showing the entire structure of a module for use in skin surgery according to a second preferred embodiment of the present invention.
FIGS. 9 to 10 are sectional conceptual views showing the entire structure of a module for use in skin surgery according to various preferred embodiments of the present invention.
FIG. 11 is a sectional conceptual view showing the entire structure of a module for use in skin surgery according to an embodiment which is not claimed.

### [Mode for Invention]

Reference will be now made in detail to the preferred embodiment of the present invention with reference to the attached drawings.

The preferred embodiments of the present invention will be described in detail with reference to the accompanying drawings so that those skilled in the art can easily implement the technical idea of the present invention, but it does not mean that the preferred embodiments do not limit the technical idea and scope of the present invention.

Moreover, it will be understood that sizes or shapes of components illustrated in the drawings may be illustrated exaggeratedly for clarity and for convenience sake in description, and it will be further understood that terms specially defined in consideration of configurations and actions of the present invention may be changed according to users' intentions or according to custom and the definition of the terms and should be interpreted in the context of the contents throughout the specification of the present invention.

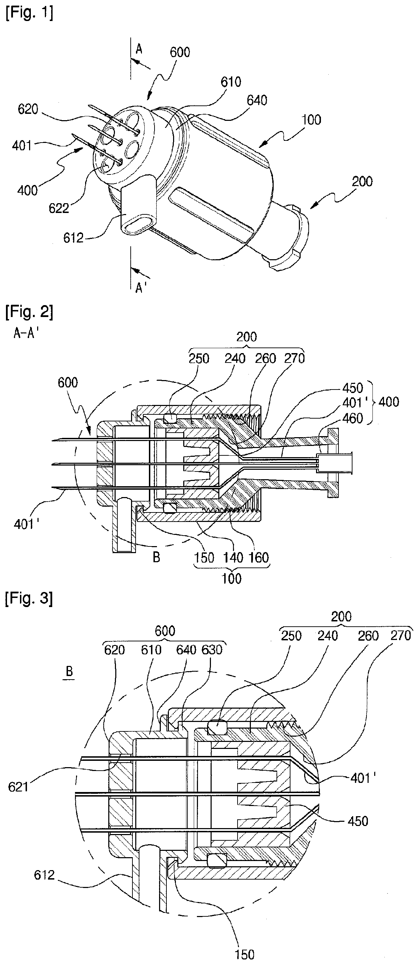

FIG. 1 is a perspective view showing an outward appearance of a module for use in skin surgery according to a first embodiment which is not claimed, FIG. 2 is a sectional view taken along the line A-A' of FIG. 1, and FIG. 3 is an enlarged view of a B part of FIG. 2.

The module for use in skin surgery according to this embodiment includes a reciprocating assembly 200 which reciprocates inside a main body 100, a needle assembly 400 mounted in the reciprocating assembly 200, and a guide suction assembly 600 for sucking air and enabling needles 401' of the needle assembly 400 to be extended and retracted.

The main body 100 has an inner space and is opened at both ends, so that the reciprocating assembly 200, the needle assembly 400 and the guide suction assembly 600 are built in the inner space.

The reciprocating assembly 200 is built in the main body 100 and screw-coupled with the main body 100 in the lengthwise direction of the main body 100 in such a way as to move forward and backward while rotating forward and backward, and provides a space for mounting the needle assembly 400 in the space.

The needle assembly 400 is built in the reciprocating assembly 200 in contact with the reciprocating assembly 200 while allowing rotation of the reciprocating assembly 200, has a plurality of needles 401' extending and retracting through an end portion of the main body 100, and forms a plurality of fine holes while the plural needles 401' extend and retract in order to promote permeation of drug into the skin.

The guide suction assembly 600 has an end portion getting in contact with the skin (not shown) and enables the end portions of the needles 401' to extend and retract through the guide suction assembly 600, and an end portion of the main body 100 is rotatably joined to the guide suction assembly 600. When the main body 100 gets in close contact with the skin according to suction of air, the guide suction assembly 600 applies suction pressure when it is necessary to gradually inject a large dose of drug into the skin by the needle assembly 400.

First, as described above, the main body 100 has the space which the reciprocating assembly 200 and the guide suction assembly 600 are built in, and includes a main tube 140, a protrusion ring 150, and a screw thread 160 as shown in FIGS. 2 and 3.

The main tube 140 is a cylindrical member, the protrusion ring 150 protrudes along the inner circumferential surface of an end portion of the main tube 140 and is joined with the guide suction assembly 600 in which the needles 401' are extended and retracted, and the screw thread 160 is formed along the inner circumferential surface of the other end portion of the main tube 140 and joined with the reciprocating assembly 200.

In the meantime, as described above, the reciprocating assembly 200 reciprocates inside the main body 100, and includes a movable body 240, a guide ring 250, a screw thread 260, and a stepped face 270 as shown in FIGS. 2 and 3.

The movable body 240 is a member opened at both ends and has a space for allowing the needle assembly 400 to be seated thereon.

The guide ring 250 protrudes along the outer circumferential surface of an end portion of the movable body 240 and gets in contact with the inner circumferential surface of the main tube 140, so as to guide the movable body 240 to carry out a reciprocating motion without any vibration while keeping a predetermined interval from the main tube 140.

The screw thread 260 is formed along the outer circumferential surface of the other end portion of the movable body 240 and joined with the screw thread 160 of the main body 140, and the stepped face 270 is steppedly formed on the inner circumferential surface of the movable body 240 and the needle assembly 400 is seated on the stepped face 270.

In the meantime, as described above, the needle assembly 400 allows rotation of the reciprocating assembly 200 in contact with the reciprocating assembly 200, and includes a body 450 in which a plurality of the needles 401' are mounted, and a distributor 460 as shown in FIG. 2.

That is, the body 450 is mounted on the stepped face 270, and is in contact with the inner circumferential surface of the movable body 240, which rotates in place, without regard to the rotation of the movable body 240.

The needles 401' are mounted in the body 450, respectively communicate with a drug injection hole (not shown) of the outside through the movable body 240, and are metallic members opened at both ends which are extended and retracted through the end portion of the guide suction assembly 600.

The distributor 460 is mounted between one end portion of each needle 401' and the drug injection hole in order to distribute drug from the drug injection hole to the needle 401', and the drug injection hole may be connected with the other end portion of the movable body 240.

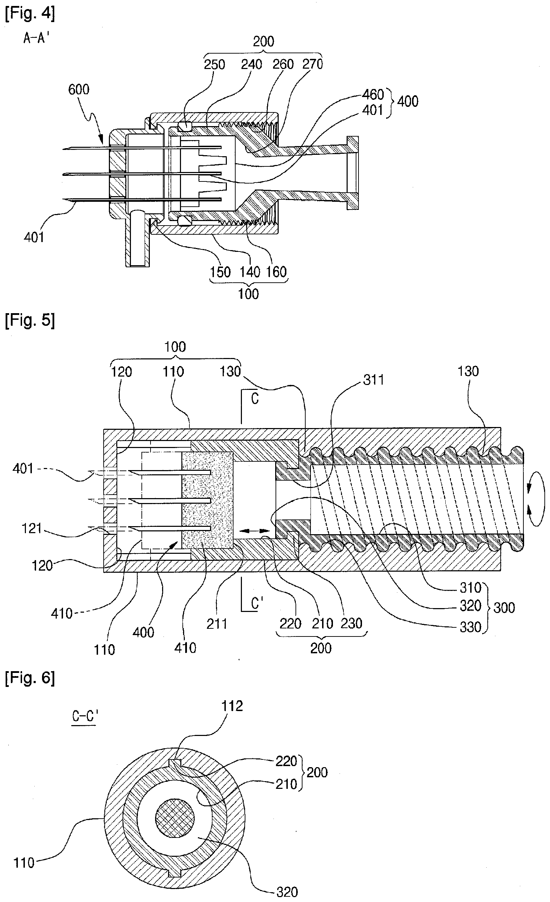

As described above, the needle assembly 400 may directly inject drug through the hollow needles 401', and alternatively, as shown in FIG. 4, in the state where needles 401 which are solid cores are mounted in the body 460, a user directly coats drug on the subject's skin and makes the needles 401 extend and retract through the main body 100 so that drug can permeate into the skin.

Meanwhile, as described above, the guide suction assembly 600 serves to guide each of the needles 401' of the needle assembly 400 and to apply suction pressure to thereby enable the user to carry out the skin surgery in close contact with the skin, and includes a body tube 610, a finishing fragment 620, a retaining ring 630, and a flange 640 as shown in FIGS. 1 to 3.

The body tube is connected with the main tube 140, and is a cylindrical member having a suction tube 612 disposed on the outer circumferential surface for sucking the inside air.

The finishing fragment 620 finishes an end portion of the body tube 610, and includes at least one suction hole 622 (See FIG. 1), through which the skin getting in contact with the guide suction assembly 400 is exposed, and through holes 621 (See FIG. 3) respectively corresponding with the needles 401'. The finishing fragment 620 provides proper tension to the skin so as to smoothly perforate holes by the needles 401'.

The retaining ring 630 protrudes along the outer circumferential surface of the other end portion of the body tube 610 and is retained to the protrusion ring 150. The flange 640 protrudes along the outer circumferential surface of the body tube 610 in parallel with the retaining ring 630, so that the protrusion ring 150 is arranged between the retaining ring 630 and the flange 640 as shown in FIG. 3 to thereby finish one end portion of the main tube 140.

Here, because the finishing fragment 620 is absorbed to the skin while air is sucked through the suction tube 612 after passing through the suction hole 622 and the body tube 610, the finishing fragment 620 absorbed to the skin provides proper tension to the skin and is usefully used when there is a need to gradually inject a large dose of drug into the skin.

Therefore, when the user rotates the main tube 140 forward and backward relative to the body tube 610 of the guide suction assembly 600, the movable body 240 screw-coupled with the inner face moves forward and backward, so that the needles 401' of the needle assembly 400 built in the movable body 240 are exposed to a proper length while extending and retracting through the guide suction assembly 600.

After that, the user contacts the finishing fragment 620 with the skin, sucks air using a device such as a suction pump (not shown) through the suction tube 612, and then, sticks the needles 401' into the skin in order to inject drug into the skin while keeping a state where the finishing fragment 620 is absorbed to the skin, such that the user can carry out the skin surgery.

Of course, another embodiment of the module for use in skin surgery, which includes a reciprocating assembly 200 reciprocating inside a main body 100, a rotation assembly 300 for rotating the reciprocating assembly 200 forward and backward, and a needle assembly 400 mounted at an end portion of the reciprocating assembly 200, is also applicable.

FIG. 5 is a sectional conceptual view showing the entire structure of a module for use in skin surgery according to a first preferred embodiment of the present invention, and FIG. 6 is a sectional view taken along the line C-C' of FIG. 5.

The main body 100 is closed at one side and opened at the other side and has an inner space which the reciprocating assembly 200, the rotation assembly 300 and the needle assembly 400 are built in.

The reciprocating assembly 200 is built in the main body 100 and reciprocates in the lengthwise direction of the main body 100. The reciprocating assembly 200 is reciprocated by the rotation assembly 300 and has a space disposed at an end portion thereof for mounting the needle assembly 400 in such a way as to be extended and retracted.

The rotation assembly 300 is rotatably joined with the reciprocating assembly 200 and is joined with the inner circumferential surface of the other end portion of the main body 100 so as to move the reciprocating assembly 200 forward and backward while rotating forward and backward.

The needle assembly 400 is mounted at an end portion of the reciprocating assembly 200 and includes a plurality of needles 401 which extend and retract through one end portion of the main body 100, and the plural needles 401 perforate a plurality of fine holes while extending and retracting through the end portion of the main body 100 in order to promote permeation of drug into the skin.

Hereinafter, the components of the module for use in skin surgery according to the present invention will be described in more detail.

As described above, the main body 100 has the space which the reciprocating assembly 200, the rotation assembly 300 and the needle assembly 400 are built in, and includes a main tube 110, a finishing fragment 120, and a screw thread 130 as shown in FIGS. 5 and 6.

The main tube 110 has at least one guide groove 112 formed in the lengthwise direction of the main body 100 for guiding the reciprocating assembly 200.

The finishing fragment 120 closes one end portion of the main tube 110 and has through holes 121 respectively corresponding with the needles 401 of the needle assembly 400.

The screw thread 130 is formed along the inner circumferential surface of the other end portion of the main tube 110 and is joined with the rotation assembly 300.

Here, the finishing fragment 120 serves to guide each of the needles 401 which are extended and retracted and to provide proper tension to the skin which comes in contact with the finishing fragment 120 so that the needles 401 can smoothly perforate holes.

In this instance, as described above, the reciprocating assembly 200 serves to extend and retract the needles 401 of the needle assembly 400 which is built in the main tube 110 of the main body 100 and mounted at the end portion while reciprocating in the lengthwise direction of the main tube 110, and includes a movable body 210, a movable protrusion 220, and a joining rib 230.

The movable body 210 is movably built in the main tube 110 and the needle assembly 400 is mounted at one end portion of the movable body 210. Referring to FIG. 6, the movable protrusion 220 will be described in more detail. The movable protrusion 220 is a part protruding from the outer circumferential surface of the movable body 210 in correspondence with the guide groove 112.

The joining rib 230 is disposed at the other end portion of the movable body 210 and is an extended part to be joined with the end portion of the rotation assembly 300 in a forward and backward rotatable manner.

In this instance, it is preferable that the movable body 210 has a stepped jaw 211 formed on the inner circumferential surface of the end portion so as to keep a state where the needle assembly 400 is seated and fixed thereon.

In the meantime, as described above, the rotation assembly 300 moves the reciprocating assembly 200 forward and backward inside the main body 100 while rotating forward and backward, and includes a rotary body 310, a retaining rib 320, and a screw thread 330.

The rotary body 310 includes a neck 311 whose outer circumference is opposed to the joining rib 230 so as to communicate with the reciprocating assembly 200 and the needle assembly 400.

The retaining rib 320 is extended from the edge of an end portion of the neck 311 and bent toward the inner circumference of the movable body 210, and is retained to the joining rib 230 so that the rotary body 310 can rotate regardless of the movable body 210.

The screw thread 330 is formed along the outer circumference of the rotary body 310 and is screw-coupled with the inner circumference of the other end portion of the main tube 110.

Accordingly, the rotation assembly 300 is crew-coupled with the main body 100 and rotates forward and backward. When the rotation assembly 300 rotates forward and backward without any influence on the reciprocating assembly 200 in the state where it is screw-coupled with the main body 100, the reciprocating assembly 200 rotatably joined with the inner circumference of the main body 100 moves forward and backward in interlock with the forward and backward rotation of the rotation assembly 300 as described above.

Meanwhile, as described above, the needle assembly is mounted in the reciprocating assembly 200 in order to perforate a plurality of fine holes into the skin while extending and retracting from the main body 100, and includes a body 410 and needles 401.

The body 410 is mounted on the stepped jaw 211 disposed at an end portion of the movable body 210 of the reciprocating assembly 200 and has a space in which a plurality of the needles 401 are mounted.

The needles 401 are solid cores mounted in the body 410 and are suitable for promoting permeation of drug into the skin after perforating fine holes into the skin to which drug is coated.

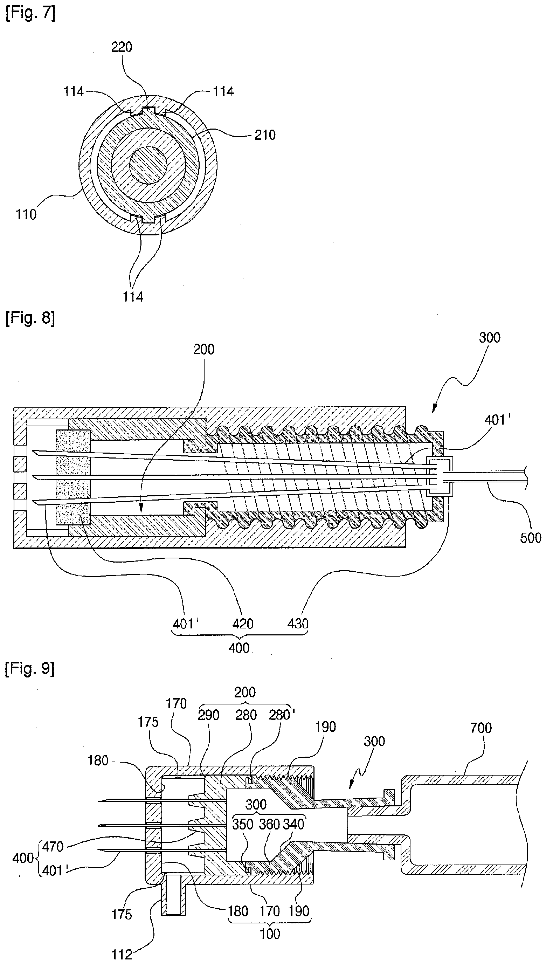

In the meantime, as shown in FIG. 7, the main body 100 and the reciprocating assembly 200 may have a structure that the movable protrusion 220 is joined between guide protrusions 114.

That is, the main body 100 may have a structure of the main tube 110 that a pair of guide protrusions 114 protrudes in parallel in the lengthwise direction of the main body 100 so as to guide the movable protrusion 220 protruding on the outer circumferential surface of the movable body 210 of the reciprocating assembly 200.

Moreover, as described above, the needle assembly 400 may adopt the solid core needles 401, but as shown in FIG. 8, may adopt hollow needles opened at both ends, or may have a structure having a main body 420, needles 401' and a distributor 430.

Because the body 420 mounted at the end portion of the reciprocating assembly 200 is nearly the same as the above embodiment, its detail description will be omitted for convenience sake.

A plurality of the needles 401' are communicated with a pipe 500 of the drug injection hole (not shown) through the reciprocating assembly 200 and the rotation assembly 300.

The distributor 430 is mounted between one end portion of each needle 401' and the drug injection hole in order to distribute drug from the pipe 500 of the drug injection hole to the respective needles 401'.

Here, the distributor 430 may keep a coupling relation by a groove and a protrusion formed at a portion getting in contact with the rotation assembly 300 so as not to be rotated integrally with the rotation assembly 300 or may adopt one of various coupling structures.

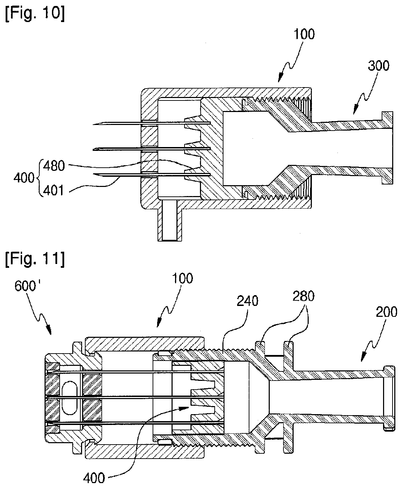

In the meantime, a suction tube 112 illustrated in FIGS. 9 and 10 may be communicatingly connected to one side of the main body 100 of the embodiment illustrated in FIGS. 5 to 8, so that the user can carry out the skin surgery while the needle assembly 400 extends and retracts in the state where the finishing fragment 180 disposed at the end portion of the main body 100 comes in contact with the skin.

That is, the main body 100 includes: the finishing fragment 180 disposed at one end portion of the main tube 170, which has a guide groove 175 formed on the inner circumferential surface in the lengthwise direction for guiding the reciprocating assembly 200, in such a manner that the needles 401' of the needle assembly 400 are respectively extended and retracted; and a screw thread 190 formed on the inner circumferential surface of the other end portion and joined with the rotation assembly 300.

Here, it is preferable that the main body 100 further includes a suction tube 112 disposed on the outer circumference of the main tube 170 of the main body 100 in such a way as to be communicated with the inside of the main tube 170 and to suck the inside air of the main tube 170.

In this instance, the suction tube 112 is to suck air in communication with the skin in the state where it comes in close contact with the skin, and when the main body 100 comes in close contact with the skin according to suction of air, suction pressure is applied when it is necessary to gradually inject a large dose of drug into the skin by the needle assembly 400.

Furthermore, the reciprocating assembly 200 is built in the main tube 170 and includes a movable body 280 disposed on the outer circumference and having a movable protrusion 290 joined to the guide groove 175, and the movable body 280 has one end portion to which the needle assembly 400 is mounted and the other end portion having a joining rib 280' joined with the end portion of the rotation assembly 300 in a forward and backward rotatable manner.

Additionally, the rotation assembly 300 includes: a rotary body 340 communicating with the reciprocating assembly 200 and the needle assembly 400; a retaining rib 350 extended along the edge of one end portion of the rotary body 340 so as to be retained to the joining rib 280'; and a screw thread 360 formed on the outer circumferential surface of the rotary body 340 and screw-coupled with the screw thread 190 of the main tube 170.

Here, the needle assembly 400 includes a plurality of needles 401' mounted at one end portion of the movable body 280; and a support fragment 470 protruding from a joined portion between the movable body 280 and the needles 401' for supporting the needles 401'.

In this instance, as shown in FIG. 9, the needle assembly 400 adopts the hollow needles 401', and is joined to the rotary body 340, which communicates with the movable body 280, so as to inject drug, which directly receives from the syringe 700, into the skin.

Moreover, as shown in FIG. 10, the needle assembly 400 may adopt the needles 401 supported by a solid core support fragment 480 and perforate fine holes into the skin while extending and retracting through the main body 100 in interlock with the forward and backward rotation of the rotation assembly 300 so as to promote permeation of drug previously coated on the skin.

Meanwhile, as shown in FIG. 11, preferably, the reciprocating assembly 200 further includes a grip flange 280 protruding along the outer circumferential surface of the movable body 240 joined with the main body 100 for the user's convenience in hand-grip so as to control the degree that the needle assembly 400 is exposed from the main body 100.

In FIG. 11, the unexplained reference numeral 600' designates a guide assembly which does not have the suction tube 612 differently from the guide suction assembly 600 of the first to fourth preferred embodiments, and of course, the embodiment of FIG 11 may adopt the guide suction assembly 600.

As described above, the module for use in skin surgery according to the present invention can accurately control a depth that drug is injected into the skin by a simple manipulation, and accurately perforate the skin while providing a predetermined tension to the skin during the skin surgery.

As described above, while the present invention has been particularly shown and described with reference to the example embodiments thereof, it will be understood by those of ordinary skill in the art that the above embodiments of the present invention are all exemplified, the drug injecting apparatus for use in skin surgery according to various embodiments may be used for use in general injection therapy, and various changes, modifications and equivalents may be made therein without departing from the scope of the present invention.

Therefore, it would be understood that the technical and protective scope of the present invention shall be defined by the technical idea as defined by the following claims.

### [Industrial Applicability]

As described above, the present invention relates to module for use in skin surgery, which can accurately control a depth that drug is injected into the skin by a simple manipulation and enables the user to perform skin surgery while providing a predetermined tension to the skin, and hence, can be used as a module for use in skin surgery for treatment of various skin troubles, such as wrinkle, freckles, blemishes, stretch marks, pimples, acnes, pigmentation, and so on, hair loss treatment, obesity treatment, etc.

## Claims

1. A module for use in skin surgery comprising:
a main body (100) closed at one side and opened at the other side and having an inner space;
a reciprocating assembly (200) built in the main body (100) and reciprocating in the lengthwise direction of the main body (100), said reciprocating assembly (200) comprising a movable body (210; 280);
a rotation assembly (300) rotatably joined with the reciprocating assembly (200) and joined with the inner circumferential surface of the other end portion of the main body (100) so as to move the reciprocating assembly (200) forward and backward while rotating forward and backward, said rotation assembly (300) comprising a rotary body (310; 340), wherein the rotary body (310; 340) can rotate regardless of the movable body (210; 280) and the reciprocating assembly (200) moves forward and backward in interlock with the forward and backward rotation of the rotation assembly (300); and
a needle assembly (400) mounted at an end portion of the reciprocating assembly (200) and having a plurality of needles (401; 401') which extend and retract through the end portion of the main body (100).

2. The module according to claim 1, wherein the main body (100) comprises:
a main tube (110; 170) having at least one guide groove (112) disposed on the inner circumferential surface in the lengthwise direction of the main body (100) for guiding the reciprocating assembly (200);
a finishing fragment (120) closing an end portion of the main tube (110; 170) and having through holes (121) respectively corresponding with the needles (401; 401') of the needle assembly (400); and
a screw thread (130) formed along the inner circumferential surface of the other end portion of the main tube (110; 170) and joined with the rotation assembly (300).

3. The module according to claim 1, wherein the main body (100) comprises:
a main tube (110; 170) having a pair of guide protrusions disposed on the inner circumferential surface in parallel in the lengthwise direction of the main body (100) for guiding the reciprocating assembly (200);
a finishing fragment (120) closing an end portion of the main tube (110; 170) and having through holes (121) respectively corresponding with the needles (401; 401') of the needle assembly (400); and
a screw thread (130) formed along the inner circumferential surface of the other end portion of the main tube (110; 170) and joined with the rotation assembly (300).

4. The module according to claim 2, wherein the reciprocating assembly (200) comprises:
a movable body (210; 280) movably built in the main tube (110; 170), to one end portion of which the needle assembly (400) is mounted;
a movable protrusion (220) protruding from the outer circumferential surface of the movable body (210; 280) in correspondence with the guide groove (112); and
a joining rib (230) disposed at the other end portion of the movable body (210; 280) and joined with the end portion of the rotation assembly (300) in a forward and backward rotatable manner.

5. The module according to claim 3, wherein the reciprocating assembly (200) comprises:
a movable body (210; 280) movably built in the main tube (110; 170), to one end portion of which the needle assembly (400) is mounted;
a movable protrusion (220) movably protruding from the outer circumferential surface of the movable body (210; 280) and joined between the guide protrusions; and
a joining rib (230) disposed at the other end portion of the movable body (210; 280) and joined with the end portion of the rotation assembly (300) in a forward and backward rotatable manner.

6. The module according to claim 4 or 5, wherein the rotation assembly (300) comprises:
a rotary body (310; 340) having a neck (311), whose outer circumference is opposed to the joining rib (230), so as to communicate with the reciprocating assembly (200) and the needle assembly (400).
a retaining rib (320) extended from the edge of an end portion of the neck (311) and bent toward the inner circumference of the movable body (210; 280); and
a screw thread (330) formed along the outer circumferential surface of the rotary body (310; 340) and screw-coupled with the inner circumferential surface of the main tube (110; 170),
wherein the rotary body (310; 340) is rotated forward and backward regardless of the movable body (210; 280).

7. The module according to claim 1, wherein the needle assembly (400) comprises:
a body (410) mounted at an end portion of the reciprocating assembly (200); and
a plurality of solid core needles (401; 401') mounted in the body

8. The module according to claim 1, wherein the needle assembly (400) comprises:
a body (410) mounted at an end portion of the reciprocating assembly (200);
a plurality of needles (401') opened at both ends, which are mounted in the body (410) and communicate with a drug injection hole of the outside through the reciprocating assembly (200) and the rotation assembly (300); and
a distributor (430) mounted between an end portion of each needle (401') and the drug injection hole for distributing drug from the drug injection hole to each needle (401').

9. The module according to claim 1, wherein the main body (100) further comprises a suction tube (112) disposed on the outer circumferential surface thereof on which the reciprocating assembly (200) reciprocates, the suction tube (112) communicating with the inside of the main body (100) for sucking the inside air of the main body (100).

10. The module according to claim 4, wherein the reciprocating assembly (200) further comprises a grip flange protruding along the outer circumference of the movable body (210; 280).

## Patentansprüche

1. Modul zur Verwendung in der Hautchirurgie, umfassend:
einen Hauptkörper (100), der an einer Seite geschlossen und an der anderen Seite geöffnet ist und einen Innenraum aufweist;
eine hin- und hergehende Baugruppe (200), die in den Hauptkörper (100) eingebaut ist und sich in der Längsrichtung des Hauptkörpers (100) hin-und herbewegt, wobei die hin- und hergehende Baugruppe (200) einen beweglichen Körper (210; 280) aufweist;
eine Rotationsbaugruppe (300), die drehbar mit der hin- und hergehenden Baugruppe (200) verbunden ist und mit der inneren Umfangsfläche des anderen Endabschnitts des Hauptkörpers (100) verbunden ist, um die hin- und
hergehende Baugruppe (200) vorwärts und rückwärts zu bewegen, während sie sich vorwärts und rückwärts rotiert, wobei die Rotationsbaugruppe (300) einen Rotationskörper (310; 340) umfasst, wobei sich der Rotationskörper (310; 340) unabhängig von dem beweglichen Körper (210; 280) drehen kann und sich die hin- und hergehende Baugruppe (200) im Zusammenspiel mit der Vorwärts- und Rückwärtsrotation der Rotationsbaugruppe (300) vorwärts und rückwärts bewegt; und
eine Nadelbaugruppe (400), die an einem Endabschnitt der hin- und hergehenden Baugruppe (200) angebracht ist und eine Vielzahl von Nadeln (401; 401') aufweist, die sich durch den Endabschnitt des Hauptkörpers (100) erstrecken und zurückziehen.

2. Modul nach Anspruch 1, wobei der Hauptkörper (100) umfasst:
ein Hauptrohr (110; 170) mit mindestens einer an der inneren Umfangsfläche in Längsrichtung des Hauptkörpers (100) angeordneten Führungsnut (112) zur Führung der hin- und hergehenden Baugruppe (200);
ein Abschlussfragment (120), das einen Endabschnitt des Hauptrohrs (110; 170) verschließt und Durchgangslöcher (121) aufweist, die jeweils den Nadeln (401; 401') der Nadelbaugruppe (400) entsprechen; und
ein Schraubgewinde (130), das entlang der inneren Umfangsfläche des anderen Endabschnitts des Hauptrohrs (110; 170) ausgebildet und mit der Rotationsbaugruppe (300) verbunden ist.

3. Modul nach Anspruch 1, wobei der Hauptkörper (100) umfasst:
ein Hauptrohr (110; 170) mit einem Paar von Führungsvorsprüngen, die an der inneren Umfangsfläche parallel in der Längsrichtung des Hauptkörpers (100) angeordnet sind, um die hin- und hergehende Baugruppe (200) zu führen;
ein Abschlussfragment (120), das einen Endabschnitt des Hauptrohrs (110; 170) verschließt und Durchgangslöcher (121) aufweist, die jeweils den Nadeln (401; 401') der Nadelbaugruppe (400) entsprechen; und
ein Schraubgewinde (130), das entlang der inneren Umfangsfläche des anderen Endabschnitts des Hauptrohrs (110; 170) ausgebildet und mit der Rotationsbaugruppe (300) verbunden ist.

4. Modul nach Anspruch 2, wobei die hin- und hergehende Baugruppe (200) umfasst:
einen beweglichen Körper (210; 280), der beweglich in das Hauptrohr (110; 170) eingebaut ist und an dessen einem Endabschnitt die Nadelbaugruppe (400) befestigt ist;
einen beweglichen Vorsprung (220), der von der äußeren Umfangsfläche des beweglichen Körpers (210; 280) in Übereinstimmung mit der Führungsnut (112) vorsteht; und
eine Verbindungsrippe (230), die an dem anderen Endabschnitt des beweglichen Körpers (210; 280) angeordnet ist und mit dem Endabschnitt der Rotationsbaugruppe (300) vorwärts und rückwärts drehbar verbunden ist.

5. Modul nach Anspruch 3, wobei die hin- und hergehende Baugruppe (200) umfasst:
einen beweglichen Körper (210; 280), der beweglich in das Hauptrohr (110; 170) eingebaut ist und an dessen einem Endabschnitt die Nadelbaugruppe (400) befestigt ist;
einen beweglichen Vorsprung (220), der von der äußeren Umfangsfläche des beweglichen Körpers (210; 280) beweglich vorsteht und zwischen den Führungsvorsprüngen verbunden ist; und
eine Verbindungsrippe (230), die an dem anderen Endabschnitt des beweglichen Körpers (210; 280) angeordnet ist und mit dem Endabschnitt der Rotationsbaugruppe (300) vorwärts und rückwärts drehbar verbunden ist.

6. Modul nach einem der Ansprüche 4 oder 5, wobei die Rotationsbaugruppe (300) umfasst:
einen Rotationskörper (310; 340) mit einem Hals (311), dessen Außenumfang der Verbindungsrippe (230) gegenüberliegt, so dass er mit der hin-und hergehenden Baugruppe (200) und der Nadelbaugruppe (400) in Verbindung steht.
eine Halterippe (320), die sich von der Kante eines Endabschnitts des Halses (311) erstreckt und in Richtung des inneren Umfangs des beweglichen Körpers (210; 280) gebogen ist; und
ein Schraubgewinde (330), das entlang der äußeren Umfangsfläche des Rotationskörpers (310; 340) ausgebildet und mit der inneren Umfangsfläche des Hauptrohrs (110; 170) verschraubt ist,
wobei der Rotationskörper (310; 340) unabhängig von dem beweglichen Körper (210; 280) vorwärts und rückwärts gerotiert wird.

7. Modul nach Anspruch 1, wobei die Nadelbaugruppe (400) umfasst:
einen Körper (410), der an einem Endabschnitt der hin- und hergehenden Baugruppe (200) angebracht ist; und
eine Vielzahl von Festkernnadeln (401; 401'), die im Körper montiert sind.

8. Modul nach Anspruch 1, wobei die Nadelbaugruppe (400) umfasst:
einen Körper (410), der an einem Endabschnitt der hin- und hergehenden Baugruppe (200) angebracht ist;
eine Vielzahl von Nadeln (401'), die an beiden Enden geöffnet sind, die in dem Körper (410) angebracht sind und mit einem Medikamenteninjektionsloch der Außenseite durch die hin- und hergehende Baugruppe (200) und die Rotationsbaugruppe (300) in Verbindung stehen; und
einen Verteiler (430), der zwischen einem Endabschnitt jeder Nadel (401') und dem Medikamenteninjektionsloch angebracht ist, um das Medikament von dem Medikamenteninjektionsloch zu jeder Nadel (401') zu verteilen.

9. Modul nach Anspruch 1, wobei der Hauptkörper (100) ferner ein Saugrohr (112) aufweist, das an seiner äußeren Umfangsfläche angeordnet ist, auf der sich die hin- und hergehende Baugruppe (200) hin- und herbewegt, wobei das Saugrohr (112) mit dem Inneren des Hauptkörpers (100) in Verbindung steht, um die Innenluft des Hauptkörpers (100) anzusaugen.

10. Modul nach Anspruch 4, wobei die hin- und hergehende Baugruppe (200) ferner einen Greifflansch aufweist, der entlang des Außenumfangs des beweglichen Körpers (210; 280) vorsteht.

## Revendications

1. Module destiné à être utilisé en chirurgie de la peau comprenant :
un corps principal (100) fermé au niveau d'un côté et ouvert au niveau de l'autre côté et ayant un espace intérieur ;
un ensemble en va-et-vient (200) intégré dans le corps principal (100) et effectuant un va-et-vient dans la direction longitudinale du corps principal (100), ledit ensemble en va-et-vient (200) comprenant un corps mobile (210 ; 280) ;
un ensemble en rotation (300) relié en rotation à l'ensemble en va-et-vient (200) et relié à la surface circonférentielle intérieure de l'autre portion d'extrémité du corps principal (100) de manière à déplacer l'ensemble en va-et-vient (200 vers l'avant et vers l'arrière tout en étant mis en rotation vers l'avant et vers l'arrière, ledit ensemble en rotation (300) comprenant un corps rotatif (310 ; 340), dans lequel le corps rotatif (310 ; 340) peut être mis en rotation indépendamment du corps mobile (210 ; 280) et l'ensemble en va-et-vient (200) se déplace vers l'avant et vers l'arrière en interverrouillage avec la rotation vers l'avant et vers l'arrière de l'ensemble en rotation (300) ; et
un ensemble à aiguilles (400) monté au niveau d'une portion d'extrémité de l'ensemble en va-et-vient (200) et ayant une pluralité d'aiguilles (401 ; 401') qui s'étendentt et se rétractent à travers la portion d'extrémité du corps principal (100).

2. Module selon la revendication 1, dans lequel le corps principal (100) comprend :
un tube principal (110 ; 170) ayant au moins une gorge de guidage (112) disposée sur la surface circonférentielle intérieure dans la direction longitudinale du corps principal (100) pour guider l'ensemble en va-et-vient (200) ;
un fragment de finition (120) fermant une partie d'extrémité du tube principal (110 ; 170) et ayant des trous de passage (121) correspondant respectivement aux aiguilles (401 ; 401') de l'ensemble à aiguilles (400) ; et un filet de vis (130) formé le long de la surface circonférentielle intérieure de l'autre partie d'extrémité du tube principal (110 ; 170) et relié à l'ensemble en rotation (300).

3. Module selon la revendication 1, dans lequel le corps principal (100) comprend :
un tube principal (110 ; 170) ayant une paire de projections de guidage disposées sur la surface circonférentielle intérieure en parallèle dans la direction longitudinale du corps principal (100) pour guider l'ensemble en va-et-vient (200) ;
un fragment de finition (120) fermant une portion d'extrémité du tube principal (110 ; 170) et ayant des trous de passage (121) correspondant respectivement aux aiguilles (401 ; 401') de l'ensemble à aiguilles (400) ; et un filet de vis (130) formé le long de la surface circonférentielle intérieure de l'autre portion d'extrémité du tube principal (110 ; 170) et relié à l'ensemble en rotation (300).

4. Module selon la revendication 2, dans lequel l'ensemble en va-et-vient (200) comprend :
un corps mobile (210 ; 280) intégré de manière mobile dans le tube principal (110 ; 170), jusqu'à une portion d'extrémité à partir de laquelle l'ensemble à aiguilles (400) est monté ;
une projection mobile (220) se projetant depuis la surface circonférentielle extérieure du corps mobile (210 ; 280) en correspondance avec la gorge de guidage (112) ; et
une nervure de liaison (230) disposée au niveau de l'autre portion d'extrémité du corps mobile (210 ; 280) et reliée à la portion d'extrémité de l'ensemble en rotation (300) d'une manière rotative vers l'avant et vers l'arrière.

5. Module selon la revendication 3, dans lequel l'ensemble en va-et-vient (200) comprend :
un corps mobile (210 ; 280) intégré de manière mobile dans le tube principal (110 ; 170) jusqu'à une portion d'extrémité à partir de laquelle l'ensemble à aiguilles (400) est monté ;
une projection mobile (220) se projetant de manière mobile depuis la surface circonférentielle extérieure du corps mobile (210 ; 280) et reliée entre les projections de guidage ; et
une nervure de liaison (230) disposée au niveau de l'autre portion d'extrémité du corps mobile (210 ; 280) et reliée à la portion d'extrémité de l'ensemble en rotation (300) d'une manière rotative vers l'avant et vers l'arrière.

6. Module selon la revendication 4 ou 5, dans lequel l'ensemble en rotation (300) comprend :
un corps rotatif (310 ; 340) ayant un étranglement (311), dont une circonférence extérieure est opposée à la nervure de liaison (230), de manière à communiquer avec l'ensemble en va-et-vient (200) et l'ensemble à aiguilles (400),
une nervure de retenue (320) étendue depuis le bord d'une portion d'extrémité de l'étranglement (311) et cintrée vers la circonférence intérieure du corps mobile (210 ; 280) ; et
un filet de vis (330) formé le long de la surface circonférentielle extérieure du corps rotatif (310 ; 340) et couplé par vissage à la surface circonférentielle intérieure du tube principal (110 ; 170),
dans lequel le corps rotatif (310 ; 340) est mis en rotation vers l'avant et vers l'arrière indépendamment du corps mobile (210 ; 280).

7. Module selon la revendication 1, dans lequel l'ensemble à aiguilles (400) comprend :
un corps (410) monté au niveau d'une portion d'extrémité de l'ensemble en va-et-vient (200) ; et
une pluralité d'aiguilles à noyau plein (401 ; 401') montées dans le corps.

8. Module selon la revendication 1, dans lequel l'ensemble à aiguilles (400) comprend :
un corps (410) monté au niveau d'une portion d'extrémité de l'ensemble en va-et-vient (200) ;
une pluralité d'aiguilles (401') ouvertes aux deux extrémités, qui sont montées dans le corps (410) et communiquent avec un trou d'injection de médicaments de l'extérieur à travers l'ensemble en va-et-vient (200) et l'ensemble en rotation (300) ; et
un distributeur (430) monté entre une portion d'extrémité de chaque aiguille (401') et le trou d'injection de médicament pour distribuer un médicament depuis le trou d'injection de médicament à chaque aiguille (401').

9. Module selon la revendication 1, dans lequel le corps principal (100) comprend en outre un tube d'aspiration (112) disposé sur la surface circonférentielle extérieure de celui-ci sur lequel l'ensemble en va-et-vient (200) effectue un va-et-vient, le tube d'aspiration (112) communiquant avec l'intérieur du corps principal (100) pour aspirer l'air à l'intérieur du corps principal (100).

10. Module selon la revendication 4, dans lequel l'ensemble en va-et-vient (200) comprend en outre une bride de préhension se projetant le long de la circonférence extérieure du corps mobile (210 ; 280).
